# EUROPEAN PATENT APPLICATION

(11) **EP 4 603 499 A1**
(43) Date of publication of application: **20.08.2025**
(21) Application number: 22962149.5
(22) Date of filing: 12.10.2022
(51) Int. Cl.: C07K 7/06, A61P 17/14, A61K 8/64, A61Q 19/00, A61K 38/00

(54) **PEPTIDE WITH ACTIVITY OF PROMOTING HAIR GROWTH AND INHIBITING HAIR LOSS AND USE THEREOF**

(30) Priority: 11.10.2022 KR 20220130080
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR); KIM, Min Woong, Anyang-si, Gyeonggi-do 14119 (KR); GIL, Hana, Anyang-si, Gyeonggi-do 14119 (KR)
(74) Representative: Heinonen & Co
(86) International application number: PCT/KR2022/015408
(87) International publication number: WO 2024/080399

(57) **Abstract**

The present invention relates to a peptide with the activity of promoting hair growth or preventing or inhibiting alopecia. The peptide of the present invention promotes the proliferation of human follicular dermal papilla cells (HFDPC), activates cell proliferation-related signaling proteins and beta-catenin in human dermal papilla cells, enhances the expression levels of beta-catenin downstream genes, down-regulates the expression levels of the hair loss protein DKK-1, increases the expression of keratin proteins in hair outer root sheath cells (HORSC) and up-regulates the expression of transcription factors involved in cell activation in germinal matrix cells (GMC).

## Description

### TECHNICAL FIELD

The present invention relates to a peptide having activities to promote hair growth and inhibit hair loss and a use thereof.

### BACKGROUND ART

Alopecia is a phenomenon in which excessive hair shedding occurs as the telogen phase becomes longer than normal or the number of hair follicles in the telogen phase increases, and means that excessive hair loss occurs due to stress, lack of nutrition, local blood flow disorders, hormonal and genetic factors, *etc*. and it cannot be restored.

As therapeutic agents for hair loss, 5α-reductase inhibitors that convert the male hormone testosterone into the androgenic sex hormone dihydrotestosterone (DHT), for examples, drugs such as finasteride or dutasteride have been developed and approved for commercial sale, and minoxidil, a drug that promotes hair growth by stimulating hair follicles and increasing blood flow, has also been approved and used.

However, in the case of 5α-reductase inhibitors, side effects such as sexual dysfunction, depression, anxiety, and breast enlargement have been reported to occur relatively frequently, and in the case of minoxidil, cardiovascular side effects such as electrocardiogram abnormalities, tachycardia, and endocarditis have been reported to occur; additionally, it has been reported that hair loss begins again when once the administration of minoxidil stops.

Peptides have high biocompatibility, and when used as a single active ingredient in drugs or cosmetics, they would have fewer side effects compared to extracts including various ingredients. After inhibiting local hair loss, these peptides can easily be decomposed and removed by proteases in the body; therefore, there is less concern about side effects of these peptide drugs compared to existing therapeutic agents.

PCT International Patent Application WO2005-082395 discloses a peptide that has the effect of promoting hair growth by promoting the progression from the telogen phase to the growth phase in the hair growth cycle, and Korea Patent No. 10-2387764 discloses a peptide derivative in a dimeric form that promotes the proliferation of hair follicle cells.

### [Prior Art Document]

### [Patent Document]

WO 2005-082395
Korea Patent No. 10-2387764

### DISCLOSURE OF THE INVENTION

### TECHNICAL PROBLEM

The present inventors have made research efforts to develop a peptide with improved activities to promote hair growth and inhibit hair loss while being effective in promoting hair growth and have fewer side effects. As a result, they have experimentally confirmed that the new peptide developed by the present inventors has the activities of promoting the proliferation of human hair follicle dermal papilla cells (HHFDPCs), activating cell proliferation-related signaling proteins, activating beta-catenin, improving the expression levels of LEF-1, c-Myc, and Cyclin D1, which are downstream factors of beta-catenin, down-regulating the expression level of hair loss protein DKK-1 in HHFDPCs, increasing the expression of keratin proteins in hair outer root sheath cells (HORSCs), and increasing the expression of transcription factors MSX2, HOXC13, and FOXN1, which are transcription factors involved in cellular activity in human hair germinal matrix cells (HHGMCs), thereby completing the present invention.

Therefore, an object of the present invention is to provide a novel peptide, which has the activities of promoting hair growth and inhibiting hair loss activities.

Another object of the present invention is to provide a composition for promoting hair growth or inhibiting, preventing, or improving alopecia, which includes the peptide having the above-described activities as an active ingredient.

Still another object of the present invention is to provide a pharmaceutical composition for preventing or treating alopecia.

Still another object of the present invention is to provide a cosmetic composition for promoting hair growth or inhibiting or improving alopecia.

### TECHNICAL SOLUTION

In order to achieve the above-identified objects,
an aspect of the present invention provides a peptide comprising the amino acid sequence of SEQ ID NO: 1.

Another aspect of the present invention provides a composition for promoting hair growth or inhibiting, preventing, or improving alopecia, which comprises the peptide as an active ingredient.

Still another aspect of the present invention provides a pharmaceutical composition for preventing or treating alopecia, which comprises the peptide as an active ingredient.

Still another aspect of the present invention provides a cosmetic composition for promoting hair growth or preventing or improving alopecia, which comprises the peptide as an active ingredient.

The present invention will be described in detail below.

### 1. Peptides and activities thereof

According to an aspect of the present invention, a peptide comprising the amino acid sequence disclosed in SEQ ID NO: 1 is provided.

As used herein, the term "peptide" refers to a linear molecule formed by linking amino acid residues to one another through peptide bonds.

The peptide comprising the amino acid sequence of SEQ ID NO: 1 of the present invention may be used without modification, but within the range that does not affect the original activity of the peptide, such as the activities of promoting hair growth or inhibiting alopecia, variants which have different amino acid sequences or a fragment thereof by deletion, insertion, substitution of amino acid residues, or a combination thereof may be used.

The peptide of the present invention may be modified by phosphorylation, sulfation, acrylation, glycosylation, methylation, farnesylation, etc., to the extent that its activities are not changed.

The peptide of the present invention includes a peptide comprising the amino acid sequence substantially identical to the peptide comprising the amino acid sequence of SEQ ID NO: 1, and a variant or active fragment thereof. The substantially identical amino acid sequence refers to an amino acid sequence having sequence identity of at least 75%, for example, at least 80%, at least 85%, at least 90%, at least 95%, and at least 97%, respectively, with the amino acid sequence of SEQ ID NO: 1. Additionally, the peptide may further include a targeting sequence, a tag, a labeled residue, and an amino acid sequence prepared for the specific purpose of increasing half-life or peptide stability.

The peptide of the present invention may have N-terminal and/or C-terminal modifications induced to select a portion of the amino acid sequence and increase its activity. Through these N-terminal and/or C-terminal modifications, the stability of the peptide of the present invention can be significantly improved, for example, the half-life of the peptide can be increased when administered *in vivo.* The term "stability" is meant to include not only *in vivo* stability, which protects the peptide of the present invention from attack by protein-cleaving enzymes *in vivo,* but also storage stability (*e.g*., storage stability at room temperature).

The N-terminal modification may be the binding, to the N-terminus of the peptide, of a protective group selected from the group consisting of an acetyl group, a fluoreonylmethoxycarbonyl group, a formyl group, a palmitoyl group, a myristyl group, a stearyl group, and polyethylene glycol (PEG). The C-terminal modification may be the binding, to the C-terminus of the peptide, of a hydroxy group (-OH), an amino group (-NH₂), an azide group (-NHNH₂), *etc.,* but is not limited thereto.

The peptide of the present invention may be prepared by various methods widely known in the technical field to which the present invention pertains. For example, the peptide of the present invention may be prepared using chemical synthesis methods known in the art, particularly solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid phase synthesis technology (U.S. Pat. No. 5,516,891).

The peptide of the present invention has the activity of promoting hair growth, or preventing or inhibiting alopecia.

In an embodiment, the peptide of the present invention promotes proliferation of hair follicle dermal papilla cells (HFDPCs).

In an embodiment, the peptide of the present invention activates Akt or ERK, which are proteins involved in signaling in hair follicle dermal papilla cells (HFDPCs).

In an embodiment, the peptide of the present invention activates beta-catenin in hair follicle dermal papilla cells (HFDPCs) and promotes its migration to the nucleus.

In an embodiment, the peptide of the present invention up-regulates the gene expression of one or more factors selected from the group consisting of LEF-1, c-Myc, and Cyclin D1, which are downstream factors of beta-catenin, in hair follicle dermal papilla cells (HFDPCs).

In an embodiment, the peptide of the present invention down-regulates the expression level of Dickkopf-1 (DKK-1), a hair loss protein, in hair follicle dermal papilla cells (HFDPCs). In hair follicles, dihydrotestosterone (DHT) promotes the expression of DKK-1, and the DKK-1, which is produced by being induced by DHT, is known to inhibit the proliferation of keratinocytes in hair follicles, to induce their apoptosis, thereby promoting hair loss (J of Invest Dermatolo. 2008, Feb., 128(2), pp. 262-269.). The expression level of DKK-1, a hair loss protein, is increased by dihydrotestosterone (DHT), and the peptide of the present invention down-regulates the expression level of DKK-1 increased by DHT.

In an embodiment, the peptide of the present invention increases the gene expression of one or more proteins selected from the group consisting of Ha3-II, Keratin 5, Keratin 14, and Keratin 19 in human hair outer root sheath cells (HHORSCs).

In an embodiment, the peptide of the present invention increases the expression of one or more transcription factors selected from the group consisting of MSX2, HOXC13, and FOXN1 in human hair germinal matrix cells (HHGMCs).

The above-mentioned peptide of the present invention can exhibit the effect of promoting hair growth, or inhibiting, preventing, or improving alopecia by the above-mentioned activities.

### 2. Composition for promoting hair growth or preventing, treating, or improving alopecia

In another aspect of the present invention, there is provided a composition for promoting hair growth, or preventing, treating, or improving alopecia, which includes a peptide comprising the amino acid sequence of SEQ ID NO: 1 as an active ingredient.

The peptide comprising the amino acid sequence of SEQ ID NO: 1 of the present invention has the activity of promoting hair growth, or inhibiting or preventing alopecia, as described above.

In another aspect of the present invention, the composition of the present invention provides a pharmaceutical composition for preventing or treating alopecia, which includes a peptide comprising the amino acid sequence of SEQ ID NO: 1 as an active ingredient.

As used herein, the term "hair loss" or "alopecia" refers to a condition, in which hair does not exist in areas where hair should normally exist due to abnormal hair loss or the number of hair is reduced compared to the normal state. In an embodiment, the hair loss or symptoms of hair loss in the present invention includes hair loss on the scalp.

In the present invention, hair loss includes hereditary androgenetic alopecia (male pattern baldness), female pattern baldness, telogen effluvium, anagen effluvium, alopecia areata, tinea capitis due to fungal infection, hypothyroidism or hair loss due to hyperthyroidism, hair loss due to inflammation (seborrheic dermatitis), hair loss due to malnutrition, hair loss due to diabetes, hair loss due to lupus, hair loss due to hair production disorders, hair loss due to medications (anticoagulants, antidepressants, discontinuation of oral contraceptives, chemotherapy), toxic folliculitis, lichen planopilaris, hair loss due to burns or trauma, hair loss due to ultraviolet rays, and hair loss due to contact with fine dust in the air, but is not limited thereto.

In an embodiment, the pharmaceutical composition, which includes the peptide of the present invention as an active ingredient, promotes the proliferation of hair follicle dermal papilla cells (HFDPCs).

In an embodiment, the pharmaceutical composition, which includes the peptide of the present invention as an active ingredient, activates Akt or ERK, a protein involved in signaling in hair follicle dermal papilla cells (HFDPCs).

In an embodiment, the pharmaceutical composition, which includes the peptide of the present invention as an active ingredient, activates beta-catenin in hair follicle dermal papilla cells (HFDPCs) and promotes its migration to the nucleus.

In an embodiment, the pharmaceutical composition, which includes the peptide of the present invention as an active ingredient, up-regulates the gene expression of one or more factors selected from the group consisting of LEF-1, c-Myc, and Cyclin D1, which are downstream factors of beta-catenin in hair follicle dermal papilla cells (HFDPCs).

In an embodiment, the pharmaceutical composition, which includes the peptide of the present invention as an active ingredient, down-regulates the expression level of Dickkopf-1 (DKK-1), a hair loss protein, in hair follicle dermal papilla cells (HFDPCs). The expression level of DKK-1, a hair loss protein, is increased by dihydrotestosterone (DHT), and the peptide of the present invention down-regulates the expression level of DKK-1 increased by DHT.

In an embodiment, the pharmaceutical composition, which includes the peptide of the present invention as an active ingredient, increases the gene expression of one or more proteins selected from the group consisting of Ha3-II, Keratin 5, Keratin 14, and Keratin 19 in human hair outer root sheath cells (HHORSCs).

In an embodiment, the pharmaceutical composition, which includes the peptide of the present invention as an active ingredient, increases the expression of one or more transcription factors selected from the group consisting of MSX2, HOXC13, and FOXN1 in human hair germinal matrix cells (HHGMCs) .

The pharmaceutical composition of the present invention may include a therapeutically effective amount of the peptide of the present invention.

The term "therapeutically effective amount" refers to an amount sufficient for the peptide, which is an active ingredient of the pharmaceutical composition of the present invention, to achieve its activity or efficacy, for example, a sufficient amount to achieve the efficacy of treating or preventing alopecia.

As used herein, the term "prevention" refers to reducing the risk of developing a disease or disorder, and refers to any actions that inhibits or delays the onset of a disease by preventing the progression of the disease or one or more clinical symptoms thereof.

As used herein, the term "treatment" refers to alleviating a disease or disorder, and includes all actions that improve or beneficially change the symptoms of a disease by arresting or reducing the progression of the disease or one or more clinical symptoms thereof.

In the present invention, the prevention or treatment of hair loss may be removing the cause of hair loss or inhibiting the progression of hair loss, or may be promoting hair growth by inhibiting hair loss or promoting hair formation.

The pharmaceutical composition of the present invention may include a pharmaceutically acceptable carrier.

The pharmaceutically acceptable carriers, which are commonly used in formulations, include lactose, dextrose, sucrose, sorbitol, mannitol, starch, gum arabic, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc., but the carriers are not limited thereto.

In addition to the above ingredients, the pharmaceutical composition of the present invention may further include lubricants, wetting agents, sweeteners, flavoring agents, emulsifiers, suspending agents, preservatives, etc., but the ingredients are not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any route suitable for treating alopecia, for example, orally or parenterally. In the case of parenteral administration, the composition may be administered by intravenous injection, subcutaneous injection, intramuscular injection, intraperitoneal injection, topical administration, or transdermal administration. Since the pharmaceutical composition of the present invention has the activity of preventing or treating alopecia, it may be applied topically, such as by applying it to the scalp.

The dose of the pharmaceutical composition may be 0.0001 µg to 100 mg, 0.001 µg to 100 mg, 0.01 µg to 100 mg, 0.1 µg to 100 mg, or 1.0 µg to 1000 mg per day, but is not limited thereto. The pharmaceutical composition may be prescribed in various ways depending on factors such as the formulation method, administration method, patient's age, weight, sex, pathological condition, food, administration time, administration route, excretion rate, and reaction sensitivity.

The pharmaceutical composition of the present invention may be prepared in unit dosage form by formulating it using a pharmaceutically acceptable carrier and/or excipient according to a method that can easily be implemented by those skilled in the art, or it may be prepared by placing it in a multi-dose container. In particular, the formulation may be in the form of a solution, suspension, or emulsion in an oil or aqueous medium, or may be in the form of an extract, powder, granule, tablet, or capsule, and may additionally include a dispersant or stabilizer.

The pharmaceutical composition of the present invention may be an external skin preparation. The external skin preparation is a preparation that can be used by applying it to the outside of the skin. When the pharmaceutical composition of the present invention is used as an external skin preparation, it may be applied to the scalp, specifically the scalp in the area where hair loss has occurred or the scalp in the area where hair growth is to be promoted. The external skin preparation may be a cream, a gel, an ointment, a skin emulsifier, a skin suspension, a transdermal delivery patch, a drug-including bandage, a lotion, or a combination thereof. The skin external preparation may be appropriately mixed as needed with ingredients commonly used in skin external preparations (*e.g.*, cosmetics and medicines), such as aqueous ingredients, oil-based ingredients, powder ingredients, alcohols, moisturizers, thickening agents, ultraviolet ray absorbers, whitening agents, preservatives, antioxidants, surfactants, fragrances, colorants, various skin nutrients, or combinations thereof. The skin external preparations may be appropriately mixed with metal sequestrants (*e.g*., disodium edetate, trisodium edetate, sodium citrate, sodium polyphosphate, sodium metaphosphate, gluconic acid, etc.), caffeine, tannin, a licorice extract, glabridin, various herbal medicines, drugs (*e.g.,* tocopherol acetate, glycyrrhizin, tranexamic acid and derivatives or salts thereof, etc.), vitamin C, magnesium ascorbate phosphate, ascorbate glucoside, arbutin, kojic acid, and saccharides (*e.g.,* glucose, fructose, trehalose, etc.).

In another aspect of the present invention, the composition of the present invention provides a cosmetic composition for promoting hair growth, or preventing or improving alopecia including a peptide, which comprises the amino acid sequence of SEQ ID NO: 1, as an active ingredient.

As used herein, the term "improvement" refers to any action that improves or benefits the symptoms of a disease or disorder.

In an embodiment, the cosmetic composition, which includes the peptide of the present invention as an active ingredient, promotes the proliferation of hair follicle dermal papilla cells (HFDPCs).

In an embodiment, the cosmetic composition, which includes the peptide of the present invention as an active ingredient, activates Akt or ERK, proteins involved in signaling in hair follicle dermal papilla cells (HFDPCs).

In an embodiment, the cosmetic composition, which includes the peptide of the present invention as an active ingredient, activates beta-catenin in hair follicle dermal papilla cells (HFDPCs) and promotes its migration to the nucleus.

In an embodiment, the cosmetic composition, which includes the peptide of the present invention as an active ingredient, up-regulates the gene expression of one or more factors selected from the group consisting of LEF-1, c-Myc, and Cyclin D1, which are downstream factors of beta-catenin in hair follicle dermal papilla cells (HFDPCs).

In an embodiment, the cosmetic composition, which includes the peptide of the present invention as an active ingredient, down-regulates the expression level of Dickkopf-1 (DKK-1), which is a hair loss protein, in hair follicle dermal papilla cells (HFDPCs). The expression level of DKK-1, the hair loss protein, is increased by dihydrotestosterone (DHT), and the peptide of the present invention down-regulates the expression level of DKK-1 increased by DHT.

In an embodiment, the cosmetic composition, which includes the peptide of the present invention as an active ingredient, increases the gene expression of one or more proteins selected from the group consisting of Ha3-II, Keratin 5, Keratin 14, and Keratin 19 in human hair outer root sheath cells (HHORSCs).

In an embodiment, the cosmetic composition, which includes the peptide of the present invention as an active ingredient, increases the expression of one or more transcription factors selected from the group consisting of MSX2, HOXC13, and FOXN1 in human hair germinal matrix cells (HHGMCs).

The cosmetic composition may be prepared in any formulation commonly prepared in the technical field to which the present invention pertains, and may be an external skin preparation. For example, the cosmetic composition may be formulated as a solution, a suspension, an emulsion, a paste, a gel, a cream, a lotion, powder, a soap, a surfactant-including cleansing, an oil, a powder foundation, an emulsion foundation, a wax foundation, a spray, etc., but is not limited thereto.

The cosmetic composition may be prepared in various forms, such as a solution (*e.g.,* a softening lotion, a nourishing lotion, a nourishing cream, a massage cream, an essence, an eye cream, a cleansing cream, a cleansing foam, a cleansing water, a pack, a spray, powder, a hair tonic, a hair cream, a hair lotion, a hair shampoo, a hair rinse, a hair conditioner, a hair spray, a hair aerosol, a pomade, a gel, *etc.*)*,* a sol-gel, an emulsion, an oil, a wax, an aerosol, *etc.,* but is not limited thereto.

The cosmetic composition of the present invention may include other additives such as excipients and carriers, and it is possible to apply and mix the usual ingredients mixed in general skin cosmetics as necessary.

When the formulation of the cosmetic composition is a paste, a cream, or a gel, it is possible to use, as a carrier ingredient, an animal oil, a vegetable oil, a wax, paraffin, starch, tragacanth, a cellulose derivative, polyethylene glycol, silicone, bentonite, silica, talc, zinc oxide, etc. may be used.

When the formulation of the cosmetic composition is powder or a spray, it is possible to use, as a carrier ingredient, lactose, talc, silica, aluminum hydroxide, calcium silicate, or polyamide powder may be used, and particularly, when the cosmetic composition is a spray, it is possible to further include a propellant, such as chlorofluorohydrocarbon, propane/butane, or dimethyl ether, but is not limited thereto.

When the cosmetic composition is in the form of a solution or emulsion, a solvent, a solubilizing agent, or an emulsifying agent, it is possible to use, as a carrier ingredient, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, sorbitan fatty acid ester, etc. may be used.

When the formulation of the cosmetic composition is a suspension, it is possible to use, as a carrier ingredient, a liquid-phase diluent (*e.g*., water, ethanol, and propylene glycol), a suspending agent (*e.g.,* ethoxylated isostearyl alcohol, polyoxyethyl sorbitol ester, polyoxyethylene sorbitan ester, *etc.*)*,* microcrystalline cellulose, aluminum metahydroxide, bentonite, agar, tragacanth, etc.

When the formulation of the cosmetic composition is a surfactant-including cleansing agent, it is possible to use, as a carrier ingredient, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamidobetaine, fatty alcohol, fatty acid glyceride, fatty acid diethanolamide, vegetable oil, a lanolin derivative, ethoxylated glycerol fatty acid ester, etc.

When the formulation of the cosmetic composition is a hair shampoo, the peptide of the present invention may be mixed with base ingredients for forming a shampoo, such as a thickener, a surfactant, a viscosity modifier, a moisturizer, a pH adjuster, a preservative, and an essential oil. CDE may be used as the thickener; LES, an anionic surfactant, and coco betaine, an amphoteric surfactant, may be used as the surfactant; polyquarter may be used as the viscosity modifier; glycerin may be used as the moisturizer; and citric acid and sodium hydroxide may be used as the pH adjuster. A grapefruit extract, etc. may be used as the preservative; additionally, essential oils of cedarwood, peppermint, and rosemary, and silk amino acids, pentaol, or vitamin E may be added.

In addition to the peptide of the present invention and carrier ingredient, the ingredients to be included in the cosmetic composition may include auxiliary ingredients commonly used in cosmetic compositions, such as antioxidants, stabilizers, solubilizers, vitamins, pigments, and fragrances, but are not limited thereto.

The peptide of the present invention may be included in the above-described composition at a concentration of 0.01 µM to 1000 µM, and specifically, the peptide of the present invention may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, or 0.05 µM to 200 µM; 0.1 µM to 800 µM, 0.1 µM to 700 µM, 0.1 µM to 600 µM, 0.1 µM to 500 µM, 0.1 µM to 300 µM, or 0.1 µM to 200 µM; 0.3 µM to 800 µM, 0.3 µM to 700 µM, 0.3 µM to 600 µM, 0.3 µM to 500 µM, 0.3 µM to 300 µM, or 0.3 µM to 200 µM; or 0.3 µM to 150 µM, 0.3 µM to 100 µM, 0.3 µM to 90 µM, 0.3 µM to 80 µM, 0.3 µM to 70 µM, or 0.3 µM to 60 µM, but is not limited thereto.

### 3. Use of peptide of present invention

In another aspect of the present invention, there is provided a use of a peptide, which comprises the amino acid sequence of SEQ ID NO: 1, for promoting hair growth, or inhibiting, preventing, treating, or improving alopecia.

In still another aspect of the present invention, there is provided a method for promoting hair growth, or inhibiting, preventing, treating or improving alopecia, which comprises administering a peptide comprising the amino acid sequence of SEQ ID NO: 1 or a composition including the peptide to a subject in need of promoting hair growth, or preventing, inhibiting, treating, or improving alopecia.

In still another aspect of the present invention, there is provided a use of the peptide comprising the amino acid sequence of SEQ ID NO: 1 for the preparation of medicament or cosmetics for promoting hair growth, or preventing, treating, or improving alopecia.

### ADVANTAGEOUS EFFECTS

The peptide of the present invention has the activities of promoting hair growth, or preventing, inhibiting, or treating alopecia.

The peptide of the present invention has the activities of promoting the proliferation of human hair follicle dermal papilla cells (HHFDPCs), activating cell proliferation-related signaling protein Akt or ERK, activating beta-catenin, enhancing the expression levels of LEF-1, c-Myc, and Cyclin D1, which are downstream genes of beta-catenin, down-regulating the expression level of hair loss protein DKK-1, increasing the expression of keratin proteins in hair outer root sheath cells (HORSCs), and increasing the expression of transcription factors MSX2, HOXC13, and FOXN1, which are transcription factors involved in cell activation in germinal matrix cells (GMCs). The peptide of the present invention can be used to promote hair growth, prevent or inhibit alopecia, or as an active ingredient for a therapeutic agent and cosmetics.

### BRIEF DESCRIPTION OF THE DRAWINGS

FIG. 1 is an experimental result showing that the peptide of the present invention promotes the proliferation of HHFDPC cells when treated at 500 nM, 5 µM, and 50 µM.
FIG. 2 is an experimental result showing that the phosphorylation of Akt and extracellular-regulated kinase (ERK), which are cell proliferation-related signaling molecules, was increased when HHFDPC cells were treated with the peptide of the present invention at 500 nM, 5 µM, and 50 µM.
FIG. 3 is an experimental result showing that when HHFDPC cells were treated with the peptide of the present invention at 500 nM, 5 µM, and 50 µM, beta-catenin was activated and its movement to the nucleus was increased.
FIG. 4 is an experimental result showing that the expression of LEF-1, Cyclin D1, and c-Myc, which are downstream factors of beta-catenin, was increased when HHFDPC cells were treated with the peptide of the present invention at 500 nM, 5 µM, and 50 µM.
FIG. 5 is an experimental result showing that when HHFDPC cells were treated with the peptide of the present invention at 500 nM, 5 µM, and 50 µM, the expression of DKK-1 (*i.e*., a hair loss protein), which was induced by DHT treatment, was reduced in a concentration-dependent manner.
FIG. 6 is an experimental result showing that when HHORSC cells were treated with the peptide of the present invention at 500 nM, 5 µM, and 50 µM, the cell activity was increased, thereby increasing the expression of cytokeratin proteins (*i.e.,* Ha3-II, Keratin 5, Keratin 14, and Keratin 19).
FIG. 7 is an experimental result showing that when HHGMC cells were treated with the peptide of the present invention at 500 nM, 5 µM, and 50 µM, the expression of transcription factors (*i.e.,* MSX2, HOXC13, and FOXN1 involved in cellular activities) was increased.

### MODE FOR CARRYING OUT THE INVENTION

Hereinafter, the present invention will be described in detail through examples. However, the following examples specifically illustrate the present invention, and the content of the present invention is not limited by the following examples.

### Preparation Example 1: Preparation of peptide and peptide complex

A peptide having the amino acid sequence of SEQ ID NO: 1 shown in Table 1 below was synthesized using an automatic peptide synthesizer (Milligen 9050, Millipore, USA), and these synthesized peptides were then purified using C18 reversed-phase high performance liquid chromatography (HPLC) (Waters Associates, USA). The column used was ACQUITY UPLC BEH300 C18 (2.1 mm × 100 mm, 1.7 µm, Waters Co, USA).

**[Table 1]**

| SEQ ID NO: | Amino acid sequence of peptide |
|---|---|
| 1 | RHVLKRR (Arg-His-Val-Leu-Lys-Arg-Arg) |

The efficacy of the peptide of SEQ ID NO: 1 prepared was evaluated through the following experiment.

### Experimental Example 1: Promoting proliferation of human hair follicle dermal papilla cells (HHFDPCs)

The effect of the peptide prepared in Preparation Example 1 above on the proliferation of human hair follicle dermal papilla cells (HHFDPCs) was tested.

HHFDPCs were seeded in a 96-well cell culture plate at a density of 4 × 10³ cells/well and cultured for 24 hours under mesenchymal stem cell complete media conditions. Then, the medium was replaced with serum-free mesenchymal stem cell medium and cultured for additional 24 hours. The cultured cell culture was treated with the peptide prepared in Preparation Example 1 above at 500 nM, 5 µM, and 50 µM, respectively, treated with 1 µM VEGF to serve as a positive control, and not treated with anything to serve as a negative control (con), and these cultures were cultured at 37°C for additional 72 hours. Thereafter, 10 µL of MTT solution (5 mg/mL) was added thereto, and the mixture was incubated in a CO₂ incubator at 37°C for 4 hours, the medium was removed, and 100 µL of DMSO was added thereto and shaken for 10 minutes. Finally, the absorbance was measured at a wavelength of 540 nm using a spectrophotometer (Molecular Devices (SpectraMax iD3, CA, USA)).

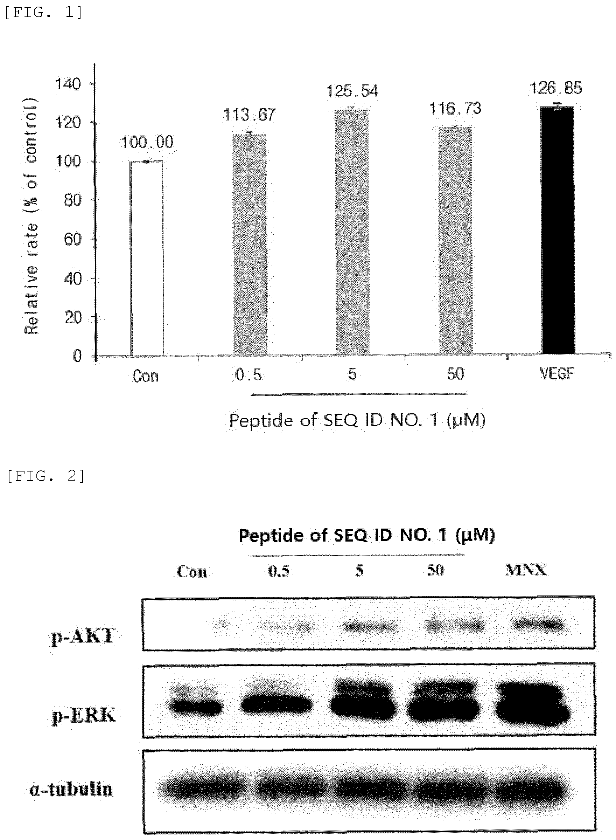

As a result of the experiment, as shown in FIG. 1, it was confirmed that the treatment of HHFDPCs with the peptide of Preparation Example 1 above at 500 nM, 5 µM, and 50 µM promoted the proliferation of HHFDPCs compared to the negative control (con).

### Experimental Example 2: Activation of proliferation-related signaling molecules in human hair follicle dermal papilla cells

The effect of the peptide prepared in Preparation Example 1 above on the activation of proliferation-related signaling molecules in human hair follicle dermal papilla cells (HHFDPCs) was confirmed by Western blot method.

HHFDPCs were seeded in a 6-well cell culture plate at a density of 4 × 10⁵ cells/well and cultured for 24 hours under mesenchymal stem cell complete media conditions. Then, the medium was replaced with serum-free mesenchymal stem cell medium and cultured for additional 24 hours. The cultured cell culture was treated with the peptide prepared in Preparation Example 1 above at 500 nM, 5 µM, and 50 µM, respectively, 1 µM of Minoxidil (MNX) was treated to serve as a positive control, and not treated with anything to serve as a negative control (con), and the cultures treated with these materials were cultured at 37°C for additional 24 hours. After washing each culture with PBS, the cells were lysed by treatment with lysis buffer to obtain a cell lysate. Equal amounts of protein samples were prepared by BCA quantification of cell lysates. The prepared protein samples were subjected to electrophoresis using 10% SDS-PAGE. The proteins separated through SDS-PAGE were transferred to a PVDF membrane and blocked at room temperature using 5% skim milk for 30 minutes. Antibodies against phospho-AKT and phospho-ERK (Cell Signaling, USA) were diluted in 3% BSA at a 1:1000 ratio and reacted with the membrane at 4°C for 16 hours. The resultant was washed 3 times for 15 minutes each with 0.1% PBS-T (0.1% Tween-20 in PBS), a secondary antibody was diluted in 5% skim milk at a 1:2000 ratio, and reacted at room temperature for 1 hour. The resultant was washed 3 times for 15 minutes each with 0.1% PBS-T (0.1% Tween-20 in PBS), treated with ECL solution (GE Healthcare, USA), and detected with a film.

As a result of the experiment, as shown in FIG. 2, it was confirmed that the treatment of HHORSCs with the peptide of Preparation Example 1 above at 500 nM, 5 µM, and 50 µM, when HHFDPCs were treated with the peptide of Preparation Example 1 above at 500 nM, 5 µM, and 50 µM, the phosphorylation of Akt and extracellular-regulated kinase (ERK) (*i.e*., cell proliferation-related signaling molecules) was increased.

### Experimental Example 3: Activation of beta-catenin in human hair follicle dermal papilla cells

The effect of the peptide prepared in Preparation Example 1 above on the activation of beta-catenin in human hair follicle dermal papilla cells (HHFDPCs) was confirmed by Western blot method.

HHFDPCs were seeded in a 6-well cell culture plate at a density of 4 × 10⁵ cells/well and cultured for 24 hours under mesenchymal stem cell complete media conditions. Then, the medium was replaced with serum-free mesenchymal stem cell medium and cultured for additional 24 hours. The cultured cell culture was treated with the peptide prepared in Preparation Example 1 above at 500 nM, 5 µM, and 50 µM, respectively, and treated with 10 ng/mL of rhWnt-3a (recombinant human Wnt-3a protein, R&D systems (MN, USA) to serve as a positive control, and not treated with anything to serve as a negative control (con). The cultures treated with these materials were incubated at 37°C for additional 24 hours. After washing each culture with PBS, nuclear proteins were isolated using a nuclear protein extraction kit (Thermo scientific, USA), and an equal amount of protein sample was prepared by BCA quantification. The prepared protein samples were subjected to electrophoresis using 10% SDS-PAGE. The proteins separated through SDS-PAGE were transferred to a PVDF membrane and blocked at room temperature for 30 minutes using 5% skim milk. An antibody against beta-catenin (Cell Signaling, USA) and an antibody against HDAC1 (Santa Cruz, USA) were diluted at a 1:1000 ratio in 3% BSA and reacted with the membrane at 4°C for 16 hours. The resultant was washed 3 times for 15 minutes each with 0.1% PBS-T (0.1% Tween-20 in PBS), a secondary antibody was diluted in 5% skim milk at a 1:2000 ratio, and reacted at room temperature for 1 hour. The resultant was washed 3 times for 15 minutes each with 0.1% PBS-T (0.1% Tween-20 in PBS), treated with ECL solution (GE Healthcare, USA), and detected with a film.

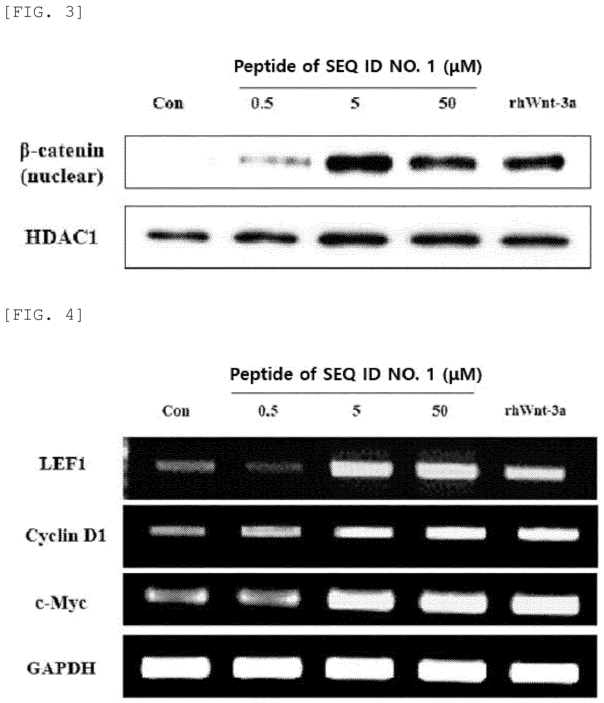

As a result of the experiment, as shown in FIG. 3, it was confirmed that when HHFDPCs were treated with the peptide of Preparation Example 1 above at 500 nM, 5 µM, and 50 µM, beta-catenin was activated and its movement to the nucleus was increased.

### Experimental Example 4: Promoting the expression of downstream genes of beta-catenin in human hair follicle dermal papilla cells

The effect of the peptide prepared in Preparation Example 1 above on the expression levels of lymphoid enhancer-binding factor 1 (LEF-1), c-Myc, and Cyclin D1, which are downstream genes of beta-catenin, in human hair follicle dermal papilla cells (HHFDPCs) was determined by the RT-PCR method.

HHFDPCs were seeded in a 6-well cell culture plate at a density of 4 × 10⁵ cells/well and cultured for 24 hours under mesenchymal stem cell complete media conditions. Then, the medium was replaced with serum free mesenchymal stem cell medium and cultured for additional 24 hours. The cultured cell culture was treated with the peptide prepared in Preparation Example 1 above at 500 nM, 5 µM, and 50 µM, respectively, and treated with 10 ng/mL of rhWnt-3a (recombinant human Wnt-3a protein, R&D systems (MN, USA) to serve as a positive control, and not treated with anything to serve as a negative control (con), and each was cultured at 37°C for additional 24 hours. After washing each culture with PBS, RNA was isolated therefrom by treatment with 300 µL of eaay blue (Intron, South Korea). The isolated RNA was quantified using Nano drop, and then subjected to cDNA synthesis using a cDNA synthesis kit (Enzynomics, South Korea). Then, a PCR reaction was performed using the primers shown in Table 2 below and a PCR premix (Enzynomics, South Korea). The PCR reaction product was electrophoresed on a 1.5% agarose gel, and the bands were detected using the Bio-Rad Gel Image System.

**[Table 2]**

| Gene | Primer | Sequence (5'->3') | SEQ ID NO: |
|---|---|---|---|
| LEF-1 | F | 5'-CCAGCTATTGTAACACCTCA-3' | 2 |
| | R | 5'-TTCAGATGTAGGCAGCTGTC-3' | 3 |
| c-Myc | F | 5'-ACCAGCAGCGACTCTGAGGAGGAAC-3' | 4 |
| | R | 5'-TGACCCTCTTGGCAGCAGGATAGTCC-3' | 5 |
| Cyclin D1 | F | 5'-ACCTTCGTTTGCCCTCTGTGCCACAGATG-3' | 6 |
| | R | 5'-AGGCCCGGAGGGCAGTCCGGGT-3' | 7 |
| GAPDH | F | 5'-GGAGCCAAAAGGGTCATCAT-3' | 8 |
| | R | 5'-GTGATGGCATGGACTGTGGT-3' | 9 |

As a result of the experiment, as shown in FIG. 4, when HHFDPCs were treated with the peptide of Preparation Example 1 above at 500 nM, 5 µM, and 50 µM, beta-catenin, which affects cell proliferation and differentiation, was activated and the gene expression of its downstream factors (*i.e.,* LEF-1, Cyclin D1, and c-Myc) was increased.

### Experimental Example 5: Inhibition of expression of hair loss protein DKK-1 in human hair follicle dermal papilla cells

It is known that in hair follicles, dihydrotestosterone (DHT) promotes the expression of Dickkopf-1 (DKK-1), and DKK-1 induced by DHT inhibits the proliferation of keratinocytes in hair follicles and causes their apoptosis, thereby promoting hair loss (J of Invest Dermatolo. 2008, Feb., 128(2), pp. 262-269.). The effect of the peptide prepared in Preparation Example 1 above on the expression level of the hair loss-inducing protein DKK-1 in human hair follicle dermal papilla cells (HHFDPCs) was confirmed by Western blot method.

HHFDPCs were seeded in a 6-well cell culture plate at a density of 4 × 10⁵ cells/well and cultured for 24 hours under mesenchymal stem cell complete media conditions. Then, the medium was replaced with serum-free mesenchymal stem cell medium and cultured for additional 24 hours. The cultured cell culture was treated with 500 nM, 5 µM, and 50 µM of the peptide prepared in Preparation Example 1 above, respectively. treated with 5 µM of finasteride to serve as a positive control, and not treated with anything to serve as a negative control group (con). In particular, DHT, an inducer of the hair loss protein DKK-1, was simultaneously treated at a concentration of 100 nM. The cell cultures treated with the above-mentioned materials were cultured at 37°C for additional 24 hours. After washing each culture with PBS, cells were lysed by treatment with lysis buffer to obtain a cell lysate. Equal amounts of protein samples were prepared by BCA quantification of cell lysates. The prepared protein samples were subjected to electrophoresis using 10% SDS-PAGE. Proteins separated through SDS-PAGE were transferred to a PVDF membrane and blocked at room temperature for 30 minutes using 5% skim milk. An antibody against DKK-1 (Cell signaling, USA) was diluted in 3% BSA at a 1:1000 ratio and then reacted with the membrane at 4°C for 16 hours. The resultant was washed 3 times for 15 minutes each with 0.1% PBS-T (0.1% Tween-20 in PBS), a secondary antibody was diluted in 5% skim milk at a 1:2000 ratio, and reacted at room temperature for 1 hour. The resultant was washed 3 times for 15 minutes each with 0.1% PBS-T (0.1% Tween-20 in PBS), treated with ECL solution (GE Healthcare, USA), and detected with a film.

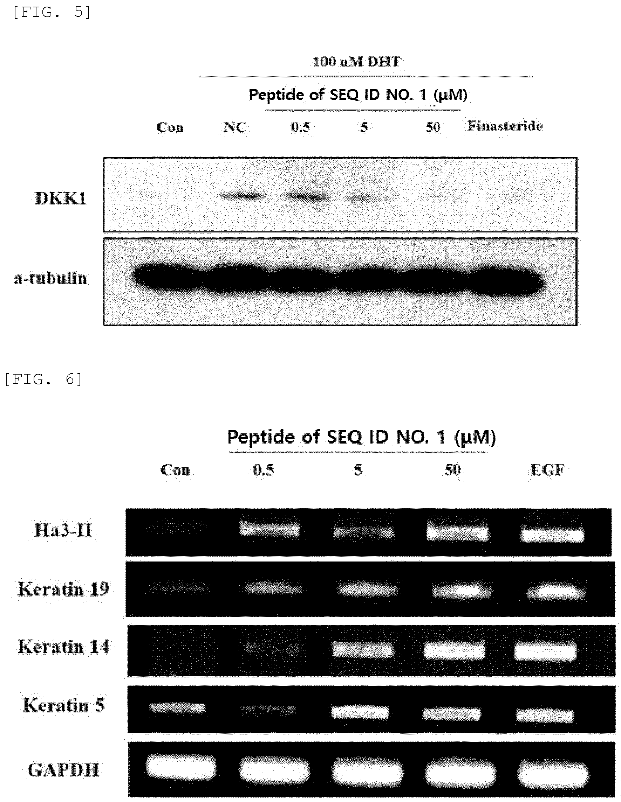

As shown in FIG. 5, it was confirmed that when HHFDPCs were treated with the peptide of Preparation Example 1 above at 500 nM, 5 µM, and 50 µM, the expression of DKK-1, a hair loss protein induced by DHT treatment, was reduced in a concentration-dependent manner.

### Experimental Example 6: Activation of human hairy outer root sheath cells (HHORSCs)

The effect of the peptide prepared in Preparation Example 1 above on the activation of human hair outer root sheath cells (HHORSCs) was confirmed by measuring the gene expression of cytokeratin proteins.

HHORSCs were seeded in a 96-well cell culture plate at a density of 4 × 10³ cells/well and cultured for 24 hours under mesenchymal stem cell complete media conditions. Then, the medium was replaced with serum free mesenchymal stem cell medium and cultured for additional 24 hours. The cultured cell culture was treated with 500 nM, 5 µM, and 50 µM of the peptide prepared in Preparation Example 1 above, respectively, and treated with 100 nM of epidermal growth factor (EGF) as a positive control, and negative when not treated with anything to serve as a negative control (con), and these cultures were cultured at 37°C for 24 hours. After washing each culture with PBS, RNA was isolated by treatment with 300 µL of eaay blue (Intron, South Korea). The isolated RNA was quantified using Nano drop, and then subjected to cDNA synthesis using a cDNA synthesis kit (Enzynomics, South Korea). Then, a PCR reaction was performed using the primers shown in Table 3 below and a PCR premix (Enzynomics, South Korea). The PCR reaction product was electrophoresed on a 1.5% agarose gel, and the bands were detected using the Bio-Rad Gel Image System.

**[Table 3]**

| Gene | Primer | Sequence (5'->3') | SEQ ID NO: |
|---|---|---|---|
| Ha3-II | F | 5'-GATCATCGAGCTGAGACGCA-3' | 10 |
| | R | 5'-CTGGCCCCAGGGTATCTAGT-3' | 11 |
| Keratin 5 | F | 5'-CCCTCAAGGATGCCAGGAAC-3' | 12 |
| | R | 5'-CACTGCTACCTCCGGCAAG-3' | 13 |
| Keratin 14 | F | 5'-CCAAATCCGCACCAAGGTCA-3' | 14 |
| | R | 5'-GTATTGATTGCCAGGAGGGGG-3' | 15 |
| Keratin 19 | F | 5'-CGCGGCGTATCCGTGTCCTC-3' | 16 |
| | R | 5'-AGCCTGTTCCGTCTCAAACTTGGT-3' | 17 |
| GAPDH | F | 5'-GGAGCCAAAAGGGTCATCAT-3' | 8 |
| | R | 5'-GTGATGGCATGGACTGTGGT-3' | 9 |

As a result of the experiment, as shown in FIG. 6, it was confirmed that when HHORSCs were treated with the peptide of Preparation Example 1 above at 500 nM, 5 µM, and 50 µM, their cell activity was increased, thereby increasing the expression of all of Ha3-II, Keratin 5, Keratin 14, and Keratin 19 (*i.e.,* cytokeratin proteins that act to form the cell skeleton and constitute hair).

### Experimental Example 7: Activation of human hair germinal matrix cells (HHGMCs)

The effect of the peptide prepared in Preparation Example 1 above on the activation of human hair germinal matrix cells (HHGMCs) was confirmed by measuring the expression of MSX2, Homeobox C13 (HOXC13), and Forkhead Box N1 (FOXN1), which are transcription factors related to cell activation.

HHGMCs were seeded in a 6-well cell culture plate at a density of 4 × 10⁵ cells/well and cultured for 24 hours under mesenchymal stem cell complete media conditions. Then, the medium was replaced with serum free mesenchymal stem cell medium and cultured for additional 24 hours. The cultured cell culture was treated with 500 nM, 5 µM, and 50 µM of the peptide prepared in Preparation Example 1 above, respectively, and treated with 100 nM of epidermal growth factor (EGF) as a positive control, and negative when not treated with anything to serve as a negative control (con), and these cultures were cultured at 37°C for 24 hours. After washing each culture with PBS, RNA was isolated by treatment with 300 µL of eaay blue (Intron, South Korea). The isolated RNA was quantified using Nano drop, and then subjected to cDNA synthesis using a cDNA synthesis kit (Enzynomics, South Korea). Then, a PCR reaction was performed using the primers shown in Table 4 below and a PCR premix (Enzynomics, South Korea). The PCR reaction product was electrophoresed on a 1.5% agarose gel, and the bands were detected using the Bio-Rad Gel Image System.

**[Table 4]**

| Gene | Primer | Sequence (5'->3') | SEQ ID NO: |
|---|---|---|---|
| MSX2 | F | 5'-CGGTCAAGTCGGAAAATTCA-3' | 18 |
| | R | 5'-GAGGAGCTGGGATGTGGTAA-3' | 19 |
| HOXC13 | F | 5'-GCCGTCTATACGGACATGCC-3' | 20 |
| | R | 5'-GCAGTACACCTGACTGTCCC-3' | 21 |
| FOXN1 | F | 5'-CAGGACTGGGTGATGGTGTC-3' | 22 |
| | R | 5'-TCTGAGGGTGAGAGCCTGAA-3' | 23 |
| GAPDH | F | 5'-GGAGCCAAAAGGGTCATCAT-3' | 8 |
| | R | 5'-GTGATGGCATGGACTGTGGT-3' | 9 |

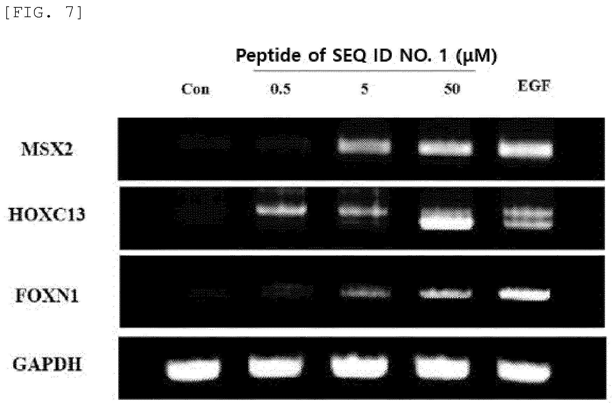

As a result of the experiment, as shown in FIG. 7, it was confirmed that when HHGMCs were treated with the peptide of Preparation Example 1 above at 500 nM, 5 µM, and 50 µM, the expression of MSX2, HOXC13, and FOXN1, which are transcription factors involved in cell activity, was increased.

### Preparation Example 2: Preparation of pharmaceutical composition

### 2-1. Preparation of powder

the peptide of the present invention 2 g
lactose 1 g

The above ingredients were mixed and filled into an airtight bubble to prepare a powder.

### 2-2. Preparation of tablets

the peptide of the present invention 100 mg
corn starch 100 mg
lactose 100 mg
magnesium stearate 2 mg

The above ingredients were mixed and prepared into tablets by tableting according to a conventional tablet preparation method.

### 2-3. Preparation of capsules

the peptide of the present invention 100 mg
corn starch 100 mg
lactose 100 mg
magnesium stearate 2 mg

The above ingredients were mixed and prepared into capsules by filling them into each gelatin capsule according to a conventional capsule preparation method.

### 2-4. Preparation of pills

the peptide of the present invention 1 g
lactose 1.5 g
glycerin 1 g
xylitol 0.5 g

The above ingredients were mixed and prepared into pills in an amount of 4 g per pill according to a conventional method.

### 2-5. Preparation of granules

the peptide of the present invention 150 mg
soybean extract 50 mg
glucose 200 mg
starch 600 mg

The above ingredients were mixed and 100 mg of 30% ethanol was added thereto and the mixture was dried at 60°C to form granules, which were then filled into pouches.

### Preparation Example 3: Preparation of cosmetic composition

### 3-1. Preparation of cream

the peptide of the present invention 4.6 parts by weight
cetostearyl alcohol 2.8 parts by weight
beeswax 2.6 parts by weight
stearic acid 1.4 parts by weight
lipophilic glycerin monostearate 2 parts by weight
PEG-100 stearate 1 part by weight
sorbital sesquioleate 1.4 parts by weight
jojoba oil 4 parts by weight
squalane 3.8 parts by weight
polysorbate 60 1.1 parts by weight
macadamia oil 2 parts by weight
tocopherol acetate 0.2 parts by weight
methylpolysiloxane 0.4 parts by weight
ethylparaben 0.1 parts by weight
propylparaben 0.1 parts by weight
Euxyl K-400 0.1 parts by weight
1,3-butylene glycol 7 parts by weight
methylparaben 0.05 parts by weight
glycerin 6 parts by weight
d-panthenol 0.2 parts by weight
triethanolamine 0.2 parts by weight
pt 41891 0.2 parts by weight
p-H₂O 46.05 parts by weight

### 3-2. Preparation of lotions

the peptide of the present invention 3.5 parts by weight
cetostearyl alcohol 1.6 parts by weight
stearic acid 1.4 parts by weight
lipophilic glycerin monostearate 1.8 parts by weight
PEG-100 stearate 2.6 parts by weight
sorbital sesquioleate 0.6 parts by weight
squalene 4.8 parts by weight
macadamia oil 2 parts by weight
jojoba oil 2 parts by weight
tocopherol acetate 0.4 parts by weight
methylpolysiloxane 0.2 parts by weight
ethylparaben 0.1 parts by weight
propylparaben 0.1 parts by weight
1,3-butylene glycol 4 parts by weight
methylparaben 0.1 parts by weight
xanthan gum 0.1 parts by weight
glycerin 4 parts by weight
d-panthenol 0.15 parts by weight
allantoin 0.1 parts by weight
calcium carbonate (2% aq. Sol) 4 parts by weight
triethanolamine 0.15 parts by weight
ethanol 3 parts by weight
pt 41891 0.1 parts by weight
p-H₂0 48.3 parts by weight

### 3-3. Preparation of softening lotions

the peptide of the present invention 0.2 wt%
ethanol 10.0 wt%
polyoxyethylene sorbitan polylaurate 1.0 wt%
methyl paraoxybenzoate 0.2 wt%
glycerin 5.0 wt%
1,3-butyl glycol 6.0 wt%
Fragrance an adequate amount
pigment an adequate amount
purified water an adequate amount
Total 100

### 3-4. Preparation of nourishing lotions

the peptide of the present invention 0.1 wt%
vaseline 2.0 wt%
sorbitan sesquioleate 0.8 wt%
polyoxyethylene oleyl ethyl 1.2 wt%
methyl paraoxybenzoate an adequate amount
propylene glycol 5.0 wt%
ethanol 3.2 wt%
carboxyvinyl polymer 18.0 wt%
pigment an adequate amount
fragrance an adequate amount
purified water an adequate amount
Total 100

### 3-5. Preparation of essences

the peptide of the present invention 5.0 wt%
propylene glycol 10.0 wt%
glycerin 10.0 wt%
an aqueous solution of sodium hyaluronate (1%) 5.0 wt%
ethanol 3.2 wt%
polyoxyethylene hydrogenated castor oil 1.0 wt%
methyl paraoxybenzoate 0.1 wt%
fragrance an adequate amount
purified water an adequate amount
Total 100

### 3-6. Preparation of packs

the peptide of the present invention 0.5 wt%
glycerin 5.0 wt%
propylene glycol 4.0 wt%
polyvinyl alcohol 15.0 wt%
ethanol 8.0 wt%
polyoxyethylene oleyl ethyl 1.0 wt%
methyl paraoxybenzoate 0.2 wt%
fragrance an adequate amount
pigment an adequate amount
purified water an adequate amount
Total 100

The above composition ratio is a mixture of suitable ingredients in preferred embodiments, but the ingredients or mixing ratios thereof may be arbitrarily modified according to regional or ethnic preferences such as demand segments, countries of demand, and intended uses.

In the above, representative embodiments of the present application have been described by way of exemplary embodiments, but the scope of the present application is not limited to the specific embodiments described above, and those skilled in the art will be able to make appropriate modifications within the scope described in the claims of this application.

## Claims

1. A peptide comprising an amino acid sequence of SEQ ID NO: 1.

2. A composition for promoting hair growth or inhibiting, preventing, or improving alopecia, comprising the peptide of claim 1 as an active ingredient.

3. A pharmaceutical composition for preventing or treating alopecia, comprising the peptide of claim 1 as an active ingredient.

4. The pharmaceutical composition of claim 3, wherein the peptide promotes the proliferation of hair follicle dermal papilla cells (HFDPCs).

5. The pharmaceutical composition of claim 3, wherein in the hair follicle dermal papilla cells (HFDPCs), the peptide:
(i) activates Akt or ERK signaling protein;
(ii) activates beta-catenin;
(iii) up-regulates the gene expression of one or more factors selected from the group consisting of LEF-1, c-Myc, and Cyclin D1, which are downstream factors of beta-catenin; or
(iv) down-regulates the expression level of Dickkopf-1 (DKK-1), a hair loss protein.

6. The pharmaceutical composition of claim 3, wherein in hair outer root sheath cells (HORSCs), the peptide increases the expression of one or more proteins selected from the group consisting of Ha3-II, Keratin 5, Keratin 14, and Keratin 19.

7. The pharmaceutical composition of claim 3, wherein in germinal matrix cells (GMCs), the peptide increases the expression of one or more transcription factors selected from the group consisting of MSX2, HOXC13, and FOXN1.

8. The pharmaceutical composition of claim 3, wherein the pharmaceutical composition is a skin external preparation.

9. A cosmetic composition for promoting hair growth or preventing or improving alopecia, comprising the peptide of claim 1 as an active ingredient.

10. The cosmetic composition of claim 9, wherein the peptide promotes the proliferation of hair follicle dermal papilla cells (HFDPCs).

11. The cosmetic composition of claim 9, wherein in the hair follicle dermal papilla cells (HFDPCs), the peptide:
(i) activates Akt or ERK signaling protein;
(ii) activates beta-catenin;
(iii) up-regulates the gene expression of one or more factors selected from the group consisting of LEF-1, c-Myc, and Cyclin D1, which are downstream factors of beta-catenin; or
(iv) down-regulates the expression level of Dickkopf-1 (DKK-1), a hair loss protein.

12. The cosmetic composition of claim 9, wherein in hair outer root sheath cells (HORSCs), the peptide increases the expression of one or more proteins selected from the group consisting of Ha3-II, Keratin 5, Keratin 14, and Keratin 19.

13. The cosmetic composition of claim 9, wherein in germinal matrix cells (GMCs), the peptide increases the expression of one or more transcription factors selected from the group consisting of MSX2, HOXC13, and FOXN1.

14. The cosmetic composition of claim 9, wherein the cosmetic composition is a skin external preparation.

15. The cosmetic composition of claim 9, wherein the cosmetic composition is one or more formulations selected from the group consisting of a solution, a suspension, an emulsion, a paste, a gel, a cream, a lotion, a powder, a soap, a surfactant-including cleansing, an oil, a powder foundation, an emulsion foundation, a wax foundation, and a spray.

16. The cosmetic composition of claim 9, wherein the cosmetic composition is one or more formulations selected from the group consisting of a hair tonic, a hair cream, a hair lotion, a hair shampoo, a hair rinse, a hair conditioner, a hair spray, a hair aerosol, a pomade, and a gel.
